# EUROPÄISCHE PATENTSCHRIFT

(11) **EP 1 044 665 B1**
(45) Veröffentlichungstag und Bekanntmachung des Hinweises auf die Patenterteilung: **02.11.2005**
(21) Anmeldenummer: 00107892.2
(22) Anmeldetag: 12.04.2000
(51) Int. Cl.: A61F 2/36

(54) **Blattartiger Schaft einer Hüftgelenkprothese für die Verankerung im Femur**
Blade-shaped stem of a hip joint prosthesis for anchoring in the femur
Tige d'une prothèse de l'articulacion de la hanche en forme de lame pour l'ancrage dans le fémur

(30) Priorität: 13.04.1999 DE 19916629; 23.06.1999 DE 19928791
(43) Veröffentlichungstag der Anmeldung: 18.10.2000
(73) Patentinhaber: PLUS ENDOPROTHETIK AG, 6343 Rotkreuz (CH)
(72) Erfinder: Zweymüller, Karl, Prof. Dr. med., 1190 Wien (AT)
(74) Vertreter: Popp, Eugen

(56) Entgegenhaltungen:
- EP-A- 0 289 922
- WO-A-00/59410
- DE-A- 2 627 569
- DE-A- 4 129 724
- DE-A- 4 223 373
- DE-U- 9 402 934
- FR-A- 2 315 902
- US-A- 3 064 645
- US-A- 5 152 799
- US-A- 5 507 833

## Beschreibung

Die Erfindung betrifft einen blattartigen Schaft einer Hüftgelenkprothese für die Verankerung im Femur mit einem Femur-Verankerungsabschnitt und einem Prothesenhals.

Derartige Profilschäfte sind allgemein bekannt. Es wird diesbezüglich nur beispielhaft verwiesen auf die EP 0 427 902 B1 oder EP 0 244 610 B1 oder US 4 908 035.

In der Regel sind die Flächen des Verankerungsabschnittes eines Schaftes der hier fraglichen Art glatt ausgebildet.

Ein solcher Schaft mit glatten Verankerungsabschnittflächen ist beispielsweise auch in der US 5 152 799 beschrieben. Der dortige Schaft verjüngt sich von einem proximalen Ende zu einem distalen Ende und hat abgerundete Kanten sowie in Längsrichtung verlaufende kanalartige Vertiefungen, um dessen Flexibilität zu erhöhen. Nachteilig bei dem in der US 5 152 799 beschriebenen Schaft ist jedoch, daß ein Einwachsen von Knochengewebe nicht oder allenfalls schlecht möglich ist, so daß die Verankerung des dortigen Schafts im Femur ungenügend und deshalb die Dauer seines Einsatzes als Schaft einer Hüftgelenkprothese relativ kurz ist. Darüber hinaus berücksichtigt dortiger Prothesenschaft keine Inhomogenitäten, insbesondere unterschiedliche Wandstärken des präparierten Femurs, so daß der Prothesenschaft insbesondere im Bereich eines dünnwandig präparierten Femurs beispielsweise im Falle vorgeschädigter Knochen, beispielsweise durch Osteoporose, bruch-, insbesondere ausbruchgefährdet ist.

In der EP 0 427 902 B1 ist vorgeschlagen, einen Abschnitt des Schaftes mit mit Sägezähnen versehenen Anlageflächen auszuführen. Dadurch soll ein Einwachsen des Schaftes in die Knochensubstanz verbessert werden.

Aus der CH-A 642 252 ist es bekannt, die nach anterior und posterior weisenden Blattseiten eines Schaftes mit rillenartigen Vertiefungen zu versehen. In diese wächst Knochengewebe jedoch nur schlecht ein. Das diese Vertiefungen ausfüllende Gewebe ist im allgemeinen ein nur wenig stabiles Bindegewebe.

Der vorliegenden Erfindung liegt insofern die Aufgabe zugrunde, den Femur-Verankerungsabschnitt eines blattartigen Schaftes so zu gestalten, daß das an die Prothese anwachsende Gewebe möglichst weitgehend aus spongiösem Knochengewebe besteht, so daß ein fester Halt des Schaftes im Femur unter Beachtung individueller Gegebenheiten des, insbesondere präparierten, Femurs dauerhaft gewährleistet ist.

Diese Aufgabe wird durch einen blattartigen Schaft mit den Merkmalen von Anspruch 1 gelöst.

Die Erfindung schließt den grundlegenden Gedanken ein, daß der Femur-Verankerungsabschnitt des Schaftes im Querschnitt im wesentlichen viereckig, also in vereinfachender (die Verjüngung zum Ende hin außer Acht lassender) Bezeichnung als "Vierkant-Profil" ausgebildet ist, und zwar insbesondere als
- Schrägkreuzprofil
- H-Profil
- Doppel-H- bzw. -Kammprofil
- Rechteck-Hohlprofil
- Rechteck-Facettenprofil
- Rechteck-Kerbprofil
- annähernd trapezförmiges Profil (mit oder ohne Ausnehmungen an den Seiten oder im Innern)
- oder dergleichen.

Bei diesen Profilen hat sich mehr oder weniger ausgeprägt gezeigt, daß sich in dem zwischen Verankerungsabschnitt des Schaftes einerseits und Operationshohlraum andererseits definierten Zwischenraum spongioses Knochengewebe bildet und damit eine Revaskularisierung des Knochen stattfindet. Die erfindungsgemäßen Alternativen haben den Vorteil, daß sie im wesentlichen vier Umrißkanten bilden, die an den Ecken eines sich senkrecht zur Schaftmittelachse erstreckenden Rechtecks oder Trapezes liegen. Diese Grundform eines Schaftes hat sich in der Praxis als besonders vorteilhaft erwiesen für die Revaskularisierung des Knochengewebes.

Weiterhin hat sich gezeigt, daß eine vorbestimmte Übermaßigkeit der Seitenflächen des Schaftes gegenüber dem Raspelmaß "(Nullmaß") - mit Ausnahme der Kantenbereiche, die paßgenau sein sollten - insbesondere im proximalen Schaftabschnitt sich in dieser Hinsicht vorteilhaft auswirkt.

Mit der erfindungsgemäßen Weiterbildung wird die Revaskularisierung des Knochengewebes zusätzlich gefördert, und zwar unter Beibehaltung der notwendigen Stabilität bzw. Festigkeit des Schaftes einerseits, jedoch unter Vergrößerung des Zwischenraumes zwischen Schaft und Operationshohlraum andererseits mit dem Ergebnis einer erhöhten Ausbildung neuer Spongiosa.

Vorteilhafte Details des erfindungsgemäßen Prothesenschaftes sind in den Unteransprüchen bzw. nachfolgend bei der Erläuterung von Ausführungsbeispielen anhand der beigefügten Zeichnungen näher erläutert. Es zeigen
- Fig. 1: eine perspektivische Ansicht eines blattartigen Schaftes, dessen Femur-Verankerungsabschnitt erfindungsgemäß weitergebildet wird;
- Fig. 2 - 9: verschiedene Querschnitte des Verankerungsabschnittes des Schaftes gemäß Fig. 1 längs der Linie A-A in Fig. 1, und
- Fig. 10: eine weitere bevorzugte Ausführung in Querschnittsdarstellung.

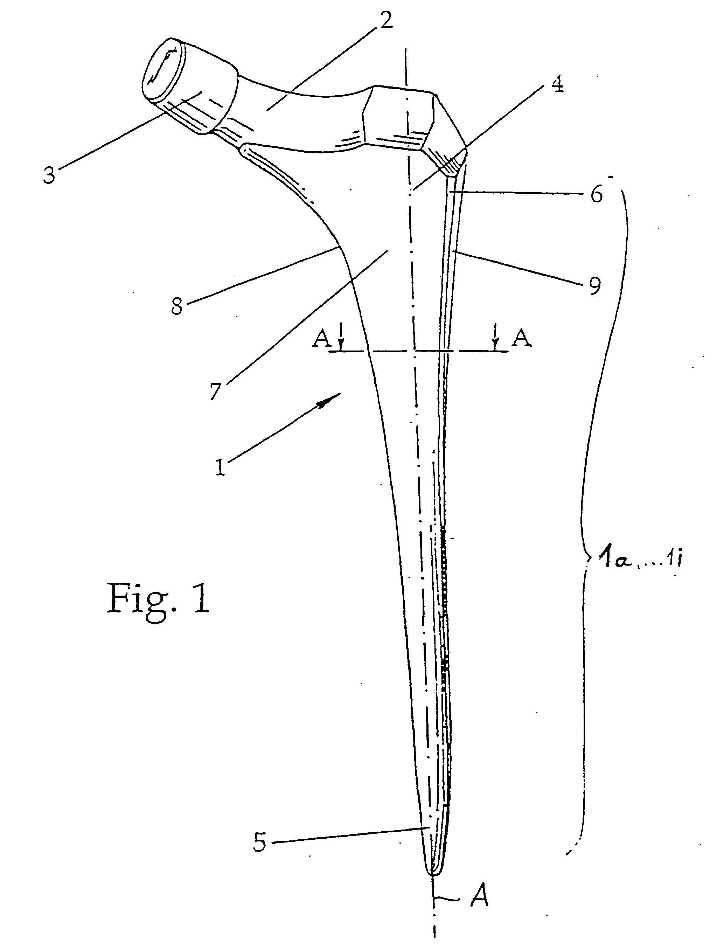

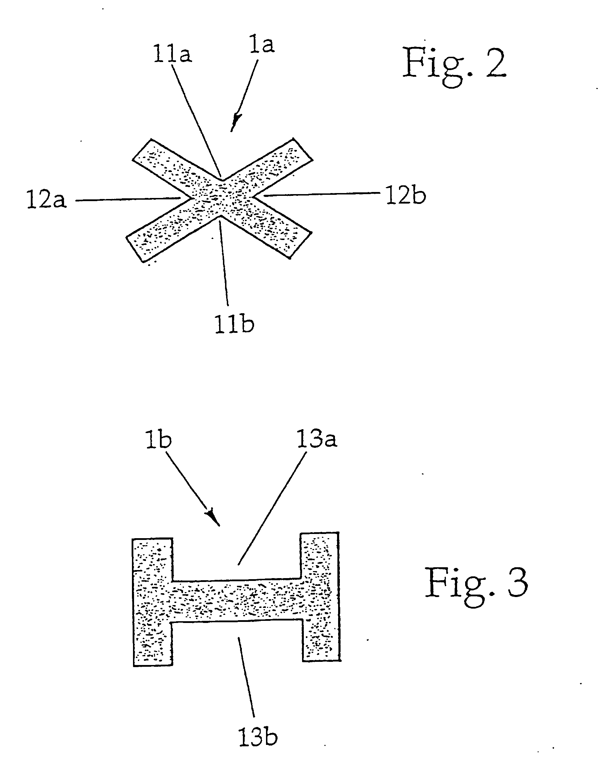

Fig. 1 zeigt in perspektivischer Ansicht einen blattartigen Schaft 1 einer Hüftgelenkprothese für die Verankerung im Femur. Das gezeigte Ausführungsbeispiel umfaßt einen sich von einem distalen Ende 5 aus nach proximal allseitig konisch erweiternden Verankerungsabschnitt 1a, ... 1i (siehe Fig. 2 bis 10), der im proximalen Bereich an der medialen Seite in einen stetig gekrümmten Bogen 8 übergeht. Dieser Bogen 8 setzt sich in einem Prothesenhals 2 fort; auf diesen ist ein sich konisch verjüngender Zapfen 3 aufgesetzt, der einen kugelförmigen Gelenkkopf aufnimmt. Die Prothesenhalsachse schneidet die (in Fig. 1 nicht dargestellte) Längsmittelachse des Schaftes bzw. Verankerungsabschnittes 1a ... 1i unter einem Winkel, der im wesentlichen dem Winkel zwischen dem Schenkelhals und der Femurachse eines natürlichen Hüftgelenkes entspricht.

Lateral ist im proximalen Bereich des Schaftes 1 ein Trochanterflügel 4 ausgebildet, der lateral durch eine Seitenfläche 9 begrenzt ist. Der Übergang zwischen der lateralen Fläche einerseits und der posterioren bzw. anterioren Fläche andererseits ist durch eine Schrägkante 6 definiert, die sich im Bereich des Trochanterflügels 4 von distal nach proximal erstreckt. Das "Blatt" des Schaftes 1 wird im proximalen Bereich definiert und ist mit der Bezugsziffer 7 gekennzeichnet.

In den Fig. 2 - 10 sind unterschiedliche Querschnitte bzw. Profilformen von Verankerungsabschnitten 1a ... 1i des Schaftes 1 dargestellt.

Gemäß Fig. 2 ist der Verankerungsabschnitt 1a als Schrägkreuzprofil ausgebildet, wobei die Schenkel anterior und posterior je eine V-förmige Nut 11a, 11b mit einem Winkel von größer als 90° und lateral und medial eine V-förmige Nut 12a, 12b mit einem Winkel von kleiner als 90° bilden.

Bei der Ausführungsform nach Fig. 3 ist der Verankerungsabschnitt 1b des Schaftes 1 als H-Profil ausgebildet. Dieses Profil umfaßt rechteckförmige Ausnehmungen 13a, 13b an der posterioren und anterioren Seite.

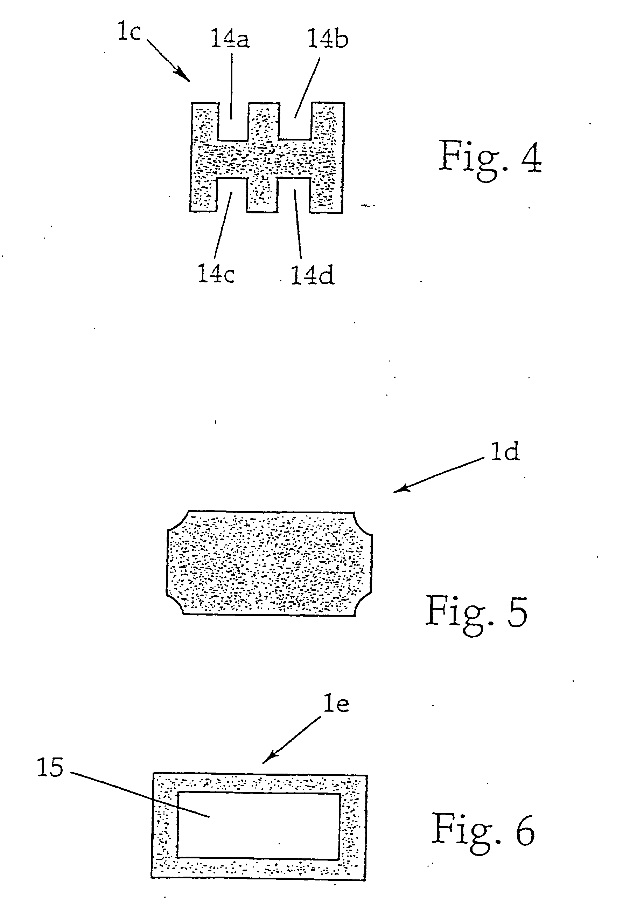

Fig. 4 zeigt eine weitere Variante, bei der der Verankerungsabschnitt 1c des Schaftes 1 ein Doppel-H-Profil bzw. Doppel-Kammprofil ist unter Ausbildung von rechteckförmigen Längsnuten 14a, 14b, 14c, 14d an der posterioren und anterioren Seite des Verankerungsabschnitts.

Bei der in Fig. 5 dargestellten Variante ist der Verankerungsabschnitt 1d des Schaftes 1 in der Querschnitts-Grobform rechteckig, mit an den vier Kanten ausgebildeten Hohlfacetten.

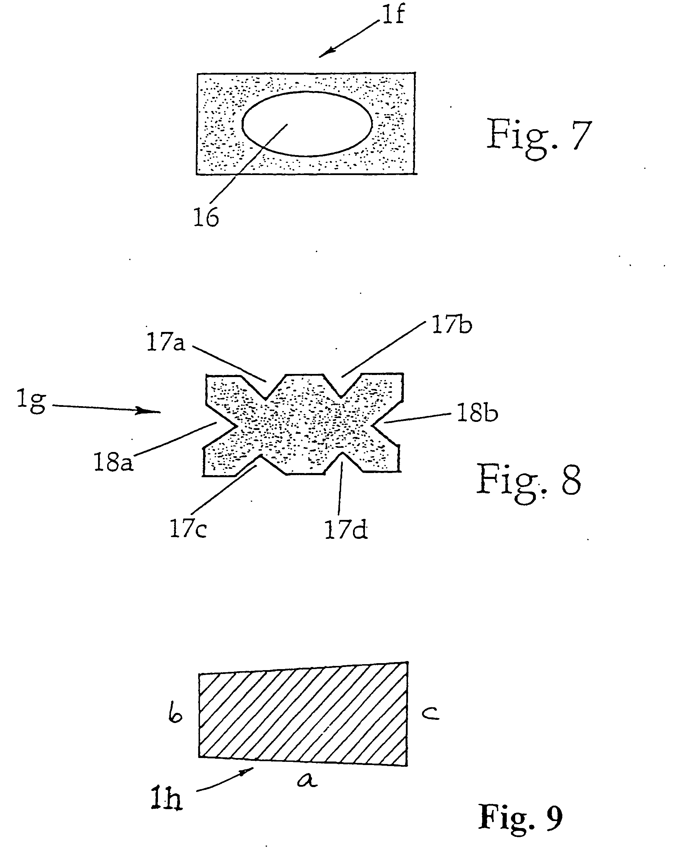

Die Ausführungsformen nach den Fig. 6 und 7 zeigen einen Verankerungsabschnitt 1e bzw. 1f in Form eines Rechteck-Hohlprofils, wobei bei der Ausführungsform nach Fig. 6 der Querschnitt des Hohlraumes 15 rechteckförmig ist, während bei der Ausführungsform nach Fig. 7 der Querschnitt des Hohlraums 16 elliptisch ist. Diese beiden Varianten zeichnen sich durch eine besonders hohe Stabilität des Verankerungsabschnittes einerseits und geringes Gewicht andererseits aus.

Die Variante gemäß Fig. 8 zeigt einen Verankerungsabschnitt 1g, der durch ein Rechteck-Kerbprofil definiert ist. Dabei sind posterior und anterior jeweils zwei voneinander beabstandete Längskerben 17a, 17b bzw. 17c, 17d ausgebildet. Es handelt sich jeweils um V-Kerben. Lateral und medial sind jeweils eine Längskerbe 18a, 18b vorgesehen, und zwar ebenfalls V-förmige Kerben bzw. Längsnuten. Die den Umriß begrenzenden Kanten des Verankerungsabschnittes 1g können ebenso wie bei der Ausführungsform nach den Fig. 6 und 7 jeweils Flach- oder Hohlfacetten entsprechend Fig. 5 aufweisen.

Bei der Ausführungsform nach Fig. 6 kann der Rechteck-Hohlraum 15 noch unterteilt sein durch einen sich in Schaftlängsrichtung erstreckenden Steg oder auch Kreuzsteg.

Die Ausführungsform nach Fig. 8 kann ebenso wie diejenige nach Fig. 5 als Hohlprofil ausgebildet sein mit einem sich in Schaftlängsrichtung erstreckenden Hohlraum, dessen Querschnitt kreisförmig oder oval bzw. elliptisch ist.

Die in Fig. 9 gezeigte Ausführung eines Verankerungsabschnitts 1h unterscheidet sich von den in den Figuren 2-8 gezeigten Ausführungen durch eine trapezförmige Querschnittsgestalt, die hier symmetrisch mit zwei gleichlangen längeren Seitenkanten a, die im Querschnitt die anterior bzw. posterior gelegenen Seitenflächen begrenzen, und zwei unterschiedlich langen kürzeren Seitenkanten b, c, von denen die kürzere medial und die längere lateral zu liegen kommt, dargestellt ist. Diese symmetrische Trapezgestalt wird derzeit als die bevorzugte angesehen, grundsätzlich sind aber auch asymmetrische trapezförmige Prothesenschaftquerschnitte ausführbar.

Auf der Grundlage der trapezförmigen Grundform sind auch Modifikationen der in Fig. 2-8 gezeigten Querschnittsgestalten (die in diesen Figuren gewissermaßen einem Rechteck einbeschrieben sind) ausführbar, also etwa ein asymmetrisches Schrägkreuz, ein "H" mit einem längeren und einem kürzeren Schenkel, eine zur Fig. 4 ähnliche Ausführung mit drei unterschiedlich langen Schenkeln, eine der Ausführung nach Fig. 5 entsprechende Ausführung mit Hohlfacetten in den Kantenbereichen eines Trapez-Querschnitts oder verschiedene Hohlprofile mit trapezförmiger Außengestalt.

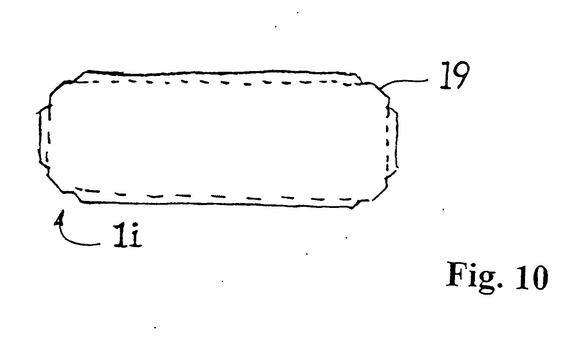

In Fig. 10 ist zur Darstellung einer weiteren speziellen Ausführung des Verankerungsabschnitts der erfindungsgemäßen Schaftprothese eine Querschnittsgestalt gezeigt, die wiederum auf der Grundform eines Rechtecks aufbaut und an allen Kanten abgestufte Anfasungsbereiche 19 aufweist. Die durch die gestrichelte Linie umschriebene Außenkontur stellt dabei skizzenhaft einen herkömmlichen Schaftquerschnitt mit unter 45° zu den Seitenflächen geneigten Anfasungsbereichen für den gleichen Einsatz dar. Es ist zu erkennen, daß die (mit einer durchgezogenen Linie gezeichnete) vorgeschlagene neue Ausführung über den größten Bereich aller Seitenflächen dieser bekannten Ausführung gegenüber übermaßig geschmiedet ist. Alle Anfasungsbereiche haben aber einen mittleren Abschnitt, der mit den Fasen des entsprechenden herkömmlichen Prothesenschaftes deckungsgleich ist. Gegenüber dieser Fase geringfügig nach innen zurückgesetzt und parallel zu ihr verlaufend sind beidseits zu den entsprechenden Seitenflächen hin jeweils Abstufungen vorgesehen.

Diese Ausführungsform geht von dem Gedanken aus, daß bei einem Prothesenschaft - mindestens im proximalen Bereich - eine vorbestimmte Übermaßigkeit gegenüber den Abmessungen des vorbereiteten Femurhohlraums (d. h. gegenüber dem "Raspelmaß") insofern vorteilhaft ist, als es die Flächenpressung gegenüber dem umgebenden Knochengewebe erhöht und dadurch eine gewisse Knochenkompression bewirkt. Das Übermaß beträgt, auch in Anbetracht der üblichen Schmiedegenauigkeit, etwa 1-3 %, bezogen auf das auch als "Nullmaß" zu verstehende Raspelmaß im Markraum.

In den Kantenbereichen hingegen soll eine möglichst hohe Paßgenauigkeit gesichert werden, um die Corticalis nicht übermäßig zu beanspruchen. Daher werden die Kantenbereiche durch nachträgliches Fräsen auf das genaue Raspelmaß gebracht.

Eine die in Fig. 10 dargestellte abgestufte Kantengestalt ergebende Nachfräsung hat sich als relativ leicht realisierbar und in vorteilhafter Weise wirkungsvoll erwiesen; grundsätzlich sind aber auch andere Feinstrukturen im Kantenbereich möglich, mit denen die Maßhaltigkeit der Kanten (genauer gesagt: der Fasen) in Einklang gebracht wird mit einer Übermaßigkeit der verbleibenden Seiten- und Stirnflächen - beispielsweise Abrundungen oder zusätzliche, gegenüber der eigentlichen Fase geneigt ausgeführte Anfasungen.

Sämtliche den Anmeldungsunterlagen offenbarten Merkmalen werden als erfindungswesentlich beansprucht, soweit sie einzeln oder in Kombination gegenüber dem Stand der Technik neu sind.

### Bezugszeichenliste

- 1: Schaft
- 1a, 1b, 1c, 1d, 1e, 1f, 1g, 1h, 1i: Femur-Verankerungsabschnitt
- 2: Prothesenhals
- 3: kegelstumpfförmiger Zapfen
- 4: Trochanterflügel
- 5: distales Ende
- 6: Facette
- 7: Abschnitt des Schaftes
- 8: Bogen
- 9: laterale Begrenzung
- 11a, 11b: V-Nut
- 12a, 12b: V-Nut
- 13a, 13b: Rechteck-Nut
- 14a, 14b, 14c, 14d: Rechteck-Nut
- 15: rechteckiger Hohlraum
- 16: ovaler Hohlraum
- 17a, 17b, 17c, 17d, 18a, 18b: Längskerbe
- 19: abgestufte Anfasung
- a: Längsseite
- b, c: Stirnseite

## Patentansprüche

1. Blattartiger Schaft (1) einer Hüftgelenkprothese für die Verankerung im Femur, mit einem sich zu einem distalen Ende (5) hin verjüngenden Femur-Verankerungsabschnitt (1a, ... 1i) mit einer Längsachse (A) sowie einem Prothesenhals (2), wobei der Femur-Verankerungsabschnitt (Ia, ... Ii) in einer Ebene senkrecht zur Längsachse (A) im wesentlichen eine viereckige insbesondere rechteckige, Außenkontur aufweist,
**dadurch gekennzeichnet,**
**daß** mindestens in einem der Längskantenbereiche des Verankerungsabschnittes (1i), insbesondere in all seinen Längskantenbereichen, eine zu den benachbarten Seitenflächen hin abgestufte, zusätzlich abgewinkelte oder abgerundete Anfasung (19) vorgesehen ist und die verbleibenden Seitenflächenbereiche mindestens in einem proximalen Teilabschnitt der Längserstreckung des Verankerungsabschnitt ein Übermaß gegenüber einem bei der Präparation des Femurs verwendeten Raspelmaß aufweisen.

2. Schaft nach Anspruch 1,
**dadurch gekennzeichnet,**
**daß** der Verankerungsabschnittes (1d - 1g; 1i) in einer Ebene senkrecht zur Längsachse (A) im wesentlichen die Form eines Rechtecks, mit Ausschnitten im Bereich mindestens einer Seitenkante und/oder der Ecken und/oder im Inneren, hat.

3. Schaft nach Anspruch 1,
**dadurch gekennzeichnet,**
**daß** der Verankerungsabschnitt (1h) in einer Ebene senkrecht zur Längsachse (A) im wesentlichen die Form eines Trapezes, insbesondere mit Ausschnitten im Bereich mindestens einer Seitenkante und/oder der Ecken und/oder im Inneren, hat.

4. Schaft nach Anspruch 3,
**dadurch gekennzeichnet,**
**daß** die Gestalt des Trapezes im wesentlichen symmetrisch ist, wobei die anteriore und posteriore Seitenkante des Trapezes gleich lang und länger als die laterale und mediale Seitenkante sind, von denen die mediale Seitenkante die kürzere ist.

5. Schaft nach einem der Ansprüche 1 - 4,
**dadurch gekennzeichnet,**
**daß** ein durch eine Ausnehmung im Inneren definierter Hohlraum (15; 16) des Verankerungsabschnittes (1e; 1f) im wesentlichen der Außenkontur des Verankerungsabschnittes angepaßt rechteckig oder trapezförmig oder aber kreisförmig oder elliptisch ausgebildet ist.

6. Schaft nach einem der Ansprüche 2 - 4,
**dadurch gekennzeichnet,**
**daß** in den die posteriore und anteriore Seitenfläche bestimmenden Seitenkanten jeweils zwei oder mehr im wesentlichen V-, U- oder C-förmige Ausnehmungen (17a - 17d) ausgebildet sind und insbesondere auch in den die laterale und/oder mediale Stirnfläche bestimmenden Begrenzungskanten jeweils wenigstens eine V-, U- oder C-förmige Ausnehmung (18a, 18b) ausgebildet ist.

7. Schaft nach einem der Ansprüche 1 - 6,
**dadurch gekennzeichnet,**
**daß** das Übermaß im Bereich zwischen 1 und 3 % liegt, wobei der Verankerungsabschnitt im übermaßigen Bereich geschmiedet ist und der Anfasungsbereich oder die Anfasungsbereiche ausgefräst ist/sind.

## Claims

1. Leaf-like shaft (1) of a hip joint prosthesis for anchoring in a femur comprising a femur-anchoring section (1a, ... 1i) tapering toward a distal end (5) having a longitudinal axis (A) as well as a prosthesis neck (2), whereby said femur-anchoring section (1a, ... 1i) exhibits an outer contour which is substantially quadrangular, particularly rectangular, in a plane perpendicular the longitudinal axis (A),
**characterized in that**
a chamfer (19) is provided in at least one of the longitudinal edge regions of the anchoring section (1i), preferably in all of its longitudinal edge regions, which has a stepped, additionally angled or rounded configuration to the adjacent side surfaces and the remaining side surface regions exhibit an overdimensioning in at least one proximal subsection of the longitudinal extension of the anchoring section with respect to the rasping dimension used in preparing the femur.

2. Shaft in accordance with claim 1, **characterized in that**
said anchoring sections (1d - 1g; 1i) have substantially the shape of a rectangle with cut-outs in a plane perpendicular to longitudinal axis (A) in the region of at least one side edge and/or corner and/or interior.

3. Shaft in accordance with claim 1, **characterized in that**
said anchoring section (1h) has substantially the shape of a trapezoid, particularly with cut-outs in a plane perpendicular to longitudinal axis (A) in the region of at least one side edge and/or corner and/or interior.

4. Shaft in accordance with claim 3, **characterized in that**
the shape of said trapezoid is substantially symmetrical whereby the anterior and posterior side edges of the trapezoid are of equal length and are longer than the lateral and medial side edges, of which the medial side edge is the shorter.

5. Shaft in accordance with one of claims 1 to 4, **characterized in that**
a cavity (15; 16) defined by a recess in the interior of anchoring section (1e; 1f) is configured in rectangular or trapezoidal or even circular or elliptical shape to substantially match the outer contour of the anchoring section.

6. Shaft in accordance with one of claims 2 to 4, **characterized in that**
two or more essentially **V**-, **U**- or **C**-shaped recesses (17a-17d) are configured at each of the side edges defining the posterior and the anterior side surface and at least one **V**-, **U**- or **C**-shaped recess (18a, 18b) is in particular also formed at each of the limiting edges defining the lateral and/or medial face surface.

7. Shaft in accordance with one of claims 1 to 6, **characterized in that**
said overdimensioning ranges between 1% and 3%, whereby the anchoring section is forged in the overdimensioned range and the chamfered region(s) is/are machined out.

## Revendications

1. Tige en forme de feuille (1) d'une prothèse d'articulation de la hanche pour l'ancrage dans le fémur, comportant une section d'ancrage de fémur (1a, ... 1i) se rétrécissant vers une extrémité distale (5), avec un axe longitudinal (A) ainsi qu'un col de prothèse (2), la section d'ancrage de fémur (1a, ... 1i) présentant un contour extérieur pour l'essentiel carré, en particulier rectangulaire, dans un plan perpendiculaire à l'axe longitudinal (A),
**caractérisée en ce que**,
au moins dans l'une des zones des arêtes longitudinales de la section d'ancrage (1i), en particulier dans toutes ses zones des arêtes longitudinales, un biseau (19), en escalier par rapport aux surfaces latérales voisines, en outre coudé ou arrondi, est prévu, et les zones de faces latérales restantes présentent dans une section partielle proximale de l'extension longitudinale de la section d'ancrage un surdimensionnement par rapport à une dimension de limage utilisée lors de la préparation du fémur.

2. Tige selon la revendication 1,
**caractérisée en ce que**
la section d'ancrage (1d à 1g ; 1i), dans un plan perpendiculaire à l'axe longitudinal (A), a pour l'essentiel la forme d'un rectangle avec des découpes dans la zone d'au moins une arête de côté et/ou des coins et/ou à l'intérieur.

3. Tige selon la revendication 1,
**caractérisée en ce que**
la section d'ancrage (1h), dans un plan perpendiculaire à l'axe longitudinal (A), a pour l'essentiel la forme d'un trapèze, en particulier avec des découpes dans la zone d'au moins une arête de côté et/ou des coins et/ou à l'intérieur.

4. Tige selon la revendication 3,
**caractérisée en ce que**
la silhouette du trapèze est pour l'essentiel symétrique, l'arête de côté antérieure et postérieure du trapèze étant de longueur égale et plus longue que l'arête de côté latérale et médiale, dont l'arête de côté médiale étant la plus courte.

5. Tige selon l'une quelconque des revendications 1 à 4,
**caractérisée en ce que**
un espace creux (15 ; 16) de la section d'ancrage (1e ; 1f) défini par un creux dans l'intérieur, est constitué, pour l'essentiel en adaptation avec le contour extérieur de la section d'ancrage, en forme de rectangle ou en forme de trapèze ou encore en forme de cercle ou en ellipse.

6. Tige selon l'une quelconque des revendications 2 à 4,
**caractérisée en ce que**
dans les arêtes de côté définissant les faces latérales postérieures et antérieures, à chaque fois deux creux (17a à 17d) ou plus sont formés, pour l'essentiel en forme de V, U ou C, et en particulier également dans les arêtes de délimitation déterminant la surface frontale latérale et/ou médiale à chaque fois au moins un creux (18a, 18b) en forme de V, U ou C est formé.

7. Tige selon l'une quelconque des revendications 1 à 6,
**caractérisée en ce que**
le surdimensionnement figure dans la plage des 1 à 3 %, la section d'ancrage dans la zone surdimensionnée étant forgée et la zone de biseau ou les zones de biseau étant fraisées.
